# EUROPÄISCHE PATENTSCHRIFT

(11) **EP 1 992 602 B1**
(45) Veröffentlichungstag und Bekanntmachung des Hinweises auf die Patenterteilung: **20.10.2010**
(21) Anmeldenummer: 08155195.4
(22) Anmeldetag: 25.04.2008
(51) Int. Cl.: C07C 17/16, C07C 19/03

(54) **Verfahren zur Herstellung von Chlormethan mit recycliertem Chlorwasserstoff**
Process for the preparation of chloromethane using recycled hydrogen chloride
Procédé pour la préparation de chlorométhane utilisant le chlorure d?hydrogène recyclé

(30) Priorität: 16.05.2007 DE 102007023052
(43) Veröffentlichungstag der Anmeldung: 19.11.2008
(73) Patentinhaber: Wacker Chemie AG, 81737 München (DE)
(72) Erfinder: Käppler, Klaus, 84489, Burghausen (DE); Nagy, Gerhard, 5144, Handenberg (AT)
(74) Vertreter: Fritz, Helmut

(56) Entgegenhaltungen:
- EP-A- 0 428 166
- DE-A1- 2 148 669
- DE-A1- 2 521 742

## Beschreibung

Die Erfindung betrifft ein Verfahren zur Herstellung von Chlormethan aus Methanol und mit Siliciumverbindungen verunreinigtem Chlorwasserstoff.

Bei der Herstellung von Chlormethan aus Methanol und Chlorwasserstoff, der aus der Methylchlorsilan-Hydrolyse stammt, werden Siliciumverbindungen in den Chlormethan-Reaktor eingebracht. Das Destillat der Chlormethan-Reaktoren enthält deshalb als unerwünschte Verunreinigung Methylchlorsilane, Methoxymethylsilane, Hydrolyse- und Kondensationsprodukte (Polymethylsiloxane) daraus, (nachstehend gemeinsam Si-Verbindungen genannt) sowie bei der Methylchlorsilansynthese (Müller-Rochow) als Nebenprodukt entstehende Kohlenwasserstoffe. Der Hauptbestandteil der Si-Verbindungen sind Polydimethylsiloxan-Cyclen, wie Hexamethylcyclotrisiloxan (D3) Octamethylcyclotetrasiloxan (D4) und Decamethylcyclopentasiloxan (D5), insbesondere D4.
Diese Si-Verbindungen können durch Kondensation nicht ausreichend aus dem Chlormethan-Gas abgeschieden werden. Ein Teil der Si-Verbindungen kondensiert im Wasserwäscher und wird zusammen mit dem ablaufenden MeOH/Wasser-Gemisch der Destillation zur Methanolrückgewinnung zugeführt. Der andere Teil der Si-Verbindungen wird zusammen mit dem MeCl aus dem Wasserwäscher ausgestrippt und anschließend bei der Trocknung des MeCl im Schwefelsäurewäscher zu Siloxanylsulfaten umgesetzt. Der Gehalt an Siloxanylsulfaten erschwert die Entsorgung oder das Recycling der Schwefelsäure, da schwer zu handhabende kieselsäureartige Feststoffe gebildet werden.
Die der destillativen Methanolrückgewinnung mit dem Methanol/Salzsäure-Gemisch zugeführten Si-Verbindungen verlassen die Kolonne zum Teil zusammen mit verdünnter Salzsäure in Form von Siloxanolen. Der andere Teil der Si-Verbindungen wird zusammen mit dem als Kopfprodukt rückgewonnenen Methanol zurück in den MeCl-Reaktor transportiert.
Über das Abwasser und vor allem über die Schwefelsäure gehen bei Siloxanproduzenten viele Jahrestonnen an Si-Verbindungen verloren. Diese Si-Verbindungen verursachen Probleme bei der Handhabung der zur Trocknung eingesetzten Schwefelsäure und belasten zusätzlich die Umwelt mit biologisch schwer abbaubaren Si-organischen Komponenten.

Die 1. Stufe der Hydrolyse von Dimethyldichlorsilan zu Polydimethylsiloxanen wird häufig mit einer im wesentlichen stöchiometrischen Wassermenge, das bedeutet mit 1 mol Wasser pro 1 mol Me₂SiCl₂, durchgeführt. Bei dieser Verfahrensweise wird das im Organochlorsilan enthaltene Chlor nicht in Form von Salzsäure, sondern in Form von weitgehend trockenem Chlorwasserstoffgas gewonnen. Die energieaufwändige Gewinnung von Chlorwasserstoff aus Salzsäure ist dann nicht notwendig.

Chlorwasserstoffgas wird benötigt zur Herstellung von MeCl, welches wiederum zur Herstellung von Methylchlorsilanen aus Si verwendet wird.

Alle Hydrolyseverfahren von Organochlorsilanen, die direkt Chlorwasserstoffgas liefern, haben jedoch den Nachteil, dass der erhaltene Chlorwasserstoff mit Siliciumverbindungen sowie Kohlenwasserstoffen verunreinigt ist.

Die Reinigung von Chlorwasserstoff, der in Chlorsilane oder Siloxane produzierenden Betrieben anfällt, und Si-Verbindungen und/oder Alkohole als Verunreinigung enthält, erfolgt im allgemeinen durch Auswaschen des Gasgemisches mit einer Waschflüssigkeit, wie Wasser, Salzsäure oder Schwefelsäure. Die destillative Reinigung von Chlorwasserstoff ist ebenfalls beschrieben.

Über die Reinigung von Chlorwasserstoff aus dem Hydrolyseprozess von Dimethyldichlorsilan, der Si-Verbindungen und Kohlenwasserstoffe enthält, wurde bisher wenig berichtet.

In einer integrierten Anlage zur Herstellung von α,ω-Dihydroxypolydimethylsiloxanen, cyclischen Polydimethylsiloxanen und MeCl bietet es sich an, α,ω-Polydimethylsiloxane als Reinigungsflüssigkeit zur Abtrennung von Si-Verbindungen aus HCl-Gas zu verwenden. Bei dieser Verfahrensweise entstehen aufgrund der Reaktivität der α,ω-Polydimethlysiloxane jedoch zahlreiche Folgeprobleme, die zu lösen sind.

Aufgrund der ungenügenden Reinheit des aus der Organochlorsilanhydrolyse stammenden Chlorwasserstoffs kann dieser nur in Flüssigphasenverfahren mit Methanol zu MeCl umgesetzt werden. Man unterscheidet katalysierte und unkatalysierte Flüssigphasenverfahren. Die Auswahl der Katalysatoren für die Flüssigphasenverfahren ist aufgrund der Verunreinigungen des Chlorwasserstoffs eingeschränkt. Katalysierte Verfahren haben jedoch den Vorteil, höherer Raum-Zeit Leistungen und höherer Ausbeuten bezüglich Methanol und HCl. Bei den in Flüssigphasenverfahren häufig eingesetzten Katalysatoren handelt es sich um Metallchloride mit Lewissäureeigenschaften wie Zinkchlorid, Eisenchlorid, Bismuthoxychlorid oder um Amine, quarternäre Ammonium- oder Phosphoniumverbindungen.

Ein Flüssigphasenverfahren zur Herstellung von MeCl unter Einsatz von Chlorwasserstoff(gas) und Methanol ist beispielsweise in EP 0 428 166 A1 beschrieben. Dort wird jedoch nicht auf die Qualität des eingesetzten Chlorwasserstoffs eingegangen.
Die über den Chlorwasserstoff eingebrachten Si-Verbindungen müssen je nach den im MeCl-Reaktor herrschenden Druck-Temperaturverhältnissen entweder in das Produkt MeCl oder über das gebildete Reaktionswasser in das Prozessabwasser gelangen. Es wird nicht beschrieben, wo die über die eingesetzte Salzsäure oder das Chlorwasserstoffgas eingeschleppten Si-Verbindungen verbleiben bzw. wie diese behandelt werden. Teilweise wurden die in der Literatur beschriebenen Verfahren nur in Laborversuchen getestet. Probleme mit in geringer Konzentration enthaltenen Si-Verbindungen werden hierbei nicht erkannt. Die Weiterverarbeitung des MeCl oder die destillative Rückgewinnung von im Überschuss eingesetztem MeOH ist nicht beschrieben.

Bei der destillativen Aufarbeitung der bei der MeCl-Synthese zwangsläufig anfallenden Gemische aus Wasser, MeOH, HCl und Si-Verbindungen polymerisieren die Si-Verbindungen häufig. Es kommt zur Verlegung in Apparaten und Rohrleitungen. Der Wärmetransport in Wärmetauschern kann behindert werden. Die im Prozessabwasser verbleibenden Si-Verbindungen sind biologisch nicht abbaubar und verursachen den so genannten persistenten CSB. Solche Si-Verbindungen sind aus Umweltgründen im Abwasser zu vermeiden.

Durch Si-Verbindungen verunreinigtes MeCl ist nicht als Rohstoff für die Direktsynthese von Organochlorsilanen aus MeCl und Si einsetzbar.

Die Abtrennung von Si-Verbindungen aus MeCl erfordert zusätzliche aufwändige Trennverfahren z. B. Destillationen. Werden die Si-Verbindungen nicht aus dem MeCl abgetrennt, verursachen sie bei der häufig angewandten Behandlung mit konzentrierter Schwefelsäure zur Abtrennung von Dimethylether (DME) und zur Trocknung Probleme durch Bildung von kieselsäureartigen Feststoffen.

Durch die in die MeC1-Synthese eingeschleppten Si-Verbindungen steigt die Prozessunsicherheit. Es ist ein Verlust von Siloxan zu verzeichnen, der die Ausbeute des Prozesses vermindert. Die Umwelt wird belastet.

Bei den sogenannten Methanolyseverfahren von Methylchlorsilanen wird das Si-gebundene Chlor durch Umsetzung mit Methanol-Wassergemischen direkt in MeCl überführt. Auch bei diesen Verfahren kann ein mit Si-Verbindungen verunreinigtes MeCl anfallen. In DE 2521742 A1 ist ein solches Verfahren beschrieben. Das den Reaktor verlassende Gemisch aus MeCl, MeOH, HCl, DME wird teilkondensiert und die nicht kondensierenden Bestandteile mit auf 12°C gekühltem Methanol gewaschen. Der Methanolwäscher dient augenscheinlich zur Abtrennung von Wasser, HCl und DME aus dem MeCl. Hinweise auf die Zusammensetzung der dem Wäscher zugeführten und abgeführten Stoffströme findet man nicht.

DE 3146526 A1 beschreibt ein Methanolyseververfahren bei dem Sx-haltiges MeCl als Dampfgemisch aus dem Reaktor abgezogen wird. Ein Teil der im MeCl enthaltenen Si-Verbindungen wird auskondensiert. Über den Restgehalt von Si-Verbindungen und die Weiterverarbeitung des MeCl wird nichts ausgesagt.

Gegenstand der Erfindung ist ein Verfahren zur Herstellung von Chlormethan aus Methanol und aus der Hydrolyse von Methylchlorsilanen stammendem mit Si-Verbindungen verunreinigtem gasförmigem Chlorwasserstoff, wobei die Si-Verbindungen ausgewählt werden aus Methylchlorsilanen, Methoxymethylsilanen und Hydrolyse- und Kondensationsprodukten daraus, bei dem aus dem gebildeten Chlormethan ein Teil der Si-Verbindungen auskondensiert werden und restliche Si-Verbindungen mit Methanol ausgewaschen werden und das dabei erhaltene Si-Verbindungen enthaltende Methanol zur Herstellung des Chlormethans mit Chlorwasserstoff eingesetzt wird und bei dem nach der teilweisen Auskondensation eines Teils der Si-Verbindungen aus dem Chlormethan eine Auftrennung des Kondensats in eine Phase der Si-Verbindungen mit organischen Verbindungen und eine Methanol-HCl-Wasserphase in einem Trennbehälter stattfindet.

Vorzugsweise wird die Umsetzung von Chlorwasserstoff mit mit Methanol zu Chlormethan in einer unter Reaktionsbedingungen flüssigen Phase durchgeführt. Vorzugsweise enthält die Flüssigphase Katalysatoren. Bevorzugte Katalysatoren sind Aminhydrochloride und die daraus unter Prozessbedingungen gebilden quaternären Methylammoniumchloride, quaternäre Phosphoniumverbindungen und Metallchloride mit Lewissäureeigenschaften, wie Zinkchlorid, Eisenchlorid, Bismuthoxychlorid.

Bei dem erfindungsgemäßen Verfahren werden vorzugsweise Hydrochloride von primären, sekundären, tertiären, linearen, cyclischen, aliphatischen und aromatischen Amine eingesetzt, mit der Maßgabe, daß die erfindungsgemäß eingesetzten Katalysatoren eine ausreichende thermische Stabilität aufweisen.

Beispiele für Amine im erfindungsgemäß eingesetzten Aminhydrochlorid sind Ammoniak, Methylamin, Trimethylamin, Diethylamin, Triethylamin, n-Butylamin, Tributylamin, Ethylendiamin, 1,4-Diazabicyclo(2.2.2)octan, 3-Dimethylaminopropylamin, Diethylentriamin, Anilin sowie mit Halogenatomen und/oder Alkylgruppen substituierte Aniline, wie N,N-Dimethylanilin, o,m,p-Phenylendiamin, Heterocyclen, wie Chinoline, Imidazole, Piperidine und Piperazine, und Pyridin sowie substituierte Pyridine, wie mit Halogenatomen, Alkyl- und/oder Aminogruppen substituierte Pyridine sowie deren quarternären Methylierungsprodukte mit MeCl.

Bevorzugte Amine im erfindungsgemäß eingesetzten Aminhydrohalogenid sind die aromatischen Amine, wie beispielsweise Aniline, Pyridine, Chinoline, Phenylendiamine sowie α- und β-Naphthylamin, wobei aromatische Amine sowie deren Methylierungsprodukte mit MeCl mit niederem Molekulargewicht besonders bevorzugt sind.

Beispiele für die im erfindungsgemäßen Verfahren besonders bevorzugt eingesetzten Hydrohalogenide sind die Hydrochloride von Pyridin, 2-Methylpyridin, 4-Methylpyridin und Anilin. Das im erfindungsgemäßen Verfahren eingesetzte Aminhydrochlorid kann als solches, beispielsweise im Gemisch mit Wasser, in den Reaktor eingeführt werden oder dort aus dem entsprechenden Amin durch Umsetzung mit Halogenwasserstoff hergestellt werden, wobei auch die Methylierungsprodukte mit MeCl entstehen.

Bei dem im erfindungsgemäßen Verfahren eingesetzten Aminhydrochlorid kann es sich um eine einzelne Art wie auch um ein Gemisch aus mindestens zwei Arten derartiger Aminhydrochloride handeln.

Vorzugsweise beträgt der Anteil an Katalysator in der Flüssigphase, bezogen auf das Gesamtgewicht der Flüssigphase 10 bis 80 Gewichtsprozent, besonders bevorzugt 35 bis 60 Gewichtsprozent, berechnet als Gewicht des freien Amins. Vorzugsweise wird Chlorwasserstoff in einer solchen Menge zugeführt, daß die Konzentration an Chlorwasserstoff in der Flüssigphase unter der jeweiligen Azeotropkonzentration liegt.

Der Flüssigphasenreaktor wird vorzugsweise bei einer Temperatur von 90 bis 200°C, besonders bevorzugt von 100 bis 180°C, und einem Druck von 900 bis 16000 hPa, besonders bevorzugt von 1000 bis 6 000 hPa, durchgeführt, wobei die Reaktionsbedingungen vorzugsweise so gewählt werden, daß das Volumen der flüssigen Phase konstant bleibt.

Bei dem erfindungsgemäßen Verfahren wird Methanol im Überschuß eingesetzt, bevorzugt 10-50 Gew. % gegenüber MeCl besonders 25-35%.

Bei dem erfindungsgemäßen Verfahren wird bevorzugt Chlorwasserstoff in solchen Mengen eingesetzt, daß die Konzentration an freiem Chlorwasserstoff in der flüssigen Phase unter der jeweiligen Azeotropkonzentration liegt. Besonders bevorzugt liegt die Konzentration an freiem Chlorwasserstoff in der Flüssigphase zwischen 0,1 und 19 Gewichtsprozent, insbesondere zwischen 0,1 und 10 Gewichtsprozent, jeweils bezogen auf das Gesamtgewicht der Flüssigphase. Als freier Chlorwasserstoff wird dabei der nicht an Amin gebundene Chlorwasserstoff bezeichnet.

Ob die Si-Verbindungen als leichtere oder schwerere Phase anfallen, kann über die Zusammensetzung der Methanol-Wasserphase gesteuert werden.

Vorzugsweise werden die abgetrennten Si-Verbindungen zurück in die Hydrolyse der Methylchlorsilane, insbesondere Dimethyldichlorsilan dosiert.

Die Löslichkeit von Si-Verbindungen in Methanol wird vom Wassergehalt beeinflußt. Deshalb beträgt der bevorzugte Wassergehalt im zum Auswaschen der Si-Verbindungen eingesetzten Methanol höchstens 10 Gew.-%, besonders bevorzugt höchstens 5 Gew.-%, insbesondere höchstens 2 Gew.-%.

Das gesamte im Verfahren eingesetzte Methanol kann zum Auswaschen der Si-Verbindungen eingesetzt werden, oder auch nur eine Teilmenge davon. Vorzugsweise werden 30 bis 100 %, insbesondere 40 bis 80 % des eingesetzten Methanols zum Auswaschen der Si-Verbindungen eingesetzt.

Vorzugsweise beträgt die Temperatur des Methanols zum Auswaschen der Si-Verbindungen 0°C bis 1°C vor dessen Siedepunkt bei dem vorherrschenden Druck.

Vorzugsweise beträgt der Druck beim Auswaschen der Si-Verbindungen mit Methanol 500 bis 5000 hPa.

Zum Auswaschen der Si-Verbindungen mit Methanol können beispielsweise Wäscher mit Füllkörpern und/oder Verteilerböden eingesetzt werden. Die Einspeisung von Methanol erfolgt vorzugsweise im oberen Drittel des Wäschers. Vorzugsweise wird Chlormethan im unteren Drittel des Wäschers zugeführt.
Zur Verbesserung der Abscheidung von mitgerissenem Methanol kann das Chlormethan durch geeignete Kühler und Abscheider zur Abtrennung von Flüssigkeitströpfchen aus Gasen geleitet und das abgeschiedene Methanol zurück in den Methanolwäscher geführt werden.

Vorzugsweise wird das aus dem Methanolwäscher entweichende Chlormethan in einem Wasserwäscher von Rest-Methanol befreit. Vorzugsweise wird das Methanol aus dem methanolhaltigen Ablauf des Wasserwäschers über eine Destillationskolonne zurückgewonnen.

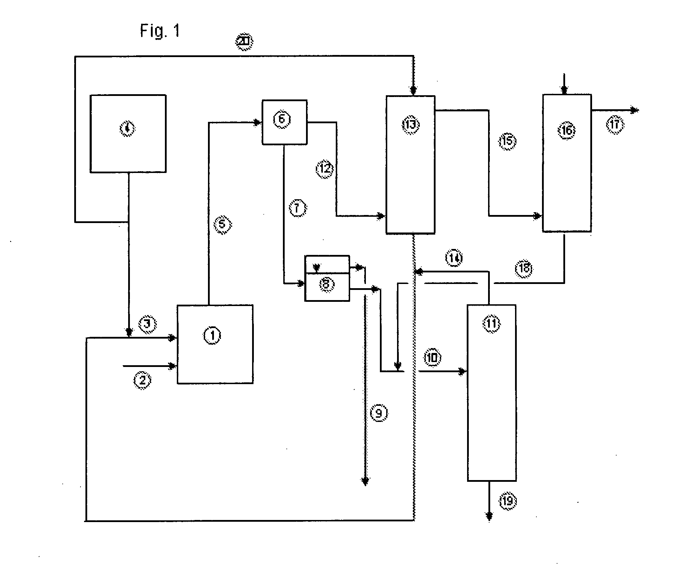

Eine bevorzugte Ausführungsform des erfindungsgemäßen Verfahrens wird anhand von Fig. 1 erläutert:

In den heizbaren Reaktor (1), der Katalysator im Gemisch mit Wasser enthält, wird über Leitung (2) Si-Verbindungen enthaltender Chlorwasserstoff und über Leitung (3) Methanol aus der Vorlage (4) und dem Methanolwäscher (13) zugespeist. Das bei der Umsetzung gebildete Chlormethan entweicht im Gemisch mit Wasser, Methanol, Si-Verbindungen und Spuren Chlorwasserstoff in den Gasraum und gelangt über Leitung (5) in den Kondensator (6), wo die Hauptmenge an Wasser, Methanol und Si-Verbindungen als Flüssigkeit abgetrennt und über Leitung (7) dem Trennbehälter (8) zugeführt werden. Im Trennbehälter (8) werden die Si-Verbindungen als Phase abgetrennt und über Leitung (9) der weiteren Verwertung zugeführt. Die Phase aus überwiegend Wasser und Methanol wird über Leitung (10) der Destillationskolonne zur Methanolrückgewinnung (11) zugeführt. Das aus dem Kondensator (6) entnommene Chlormethan gelangt über Leitung (12) in den Methanolwäscher (13), wo es im Gegenstrom mit frischem Methanol aus Leitung (20) von Si-Verbindungen befreit wird. Das aus dem Methanolwäscher (13) ablaufende Methanol, welches Si-Verbindungen enthält, wird gemeinsam mit dem in der Destillationskolonne (11) rückgewonnenen und über Leitung (14) zurückgeführten Methanol über Leitung (3) in den Reaktor (1) eingespeist. Das aus dem Methanolwäscher (13) entweichende Chlormethan gelangt über Leitung (15) in den Wasserwäscher (16), einem Apparat, in der das Chlormethan durch Kontaktierung mit von am Kopf eingeführtem Wasser von Methanol befreit wird, und verläßt über Leitung (17) die Anlage. Das methanolhaltige Wasser aus dem Wasserwäscher (16) wird über Leitung (18) der Destillationskolonne (11) zugeführt. Das im Sumpf der Destillationskolonne (11) enthaltene Wasser verläßt über Leitung (19) die Anlage.

In den folgenden Beispielen und Vergleichsbeispielen sind, falls jeweils nicht anders angegeben, alle Mengen- und Prozentangaben auf das Gewicht bezogen und sämtliche Umsetzungen werden bei einem Druck von 0,10 MPa (abs.) und einer Temperatur von 20°C durchgeführt.

### Beispiele

### Nicht erfindungsgemäßes Beispiel 1

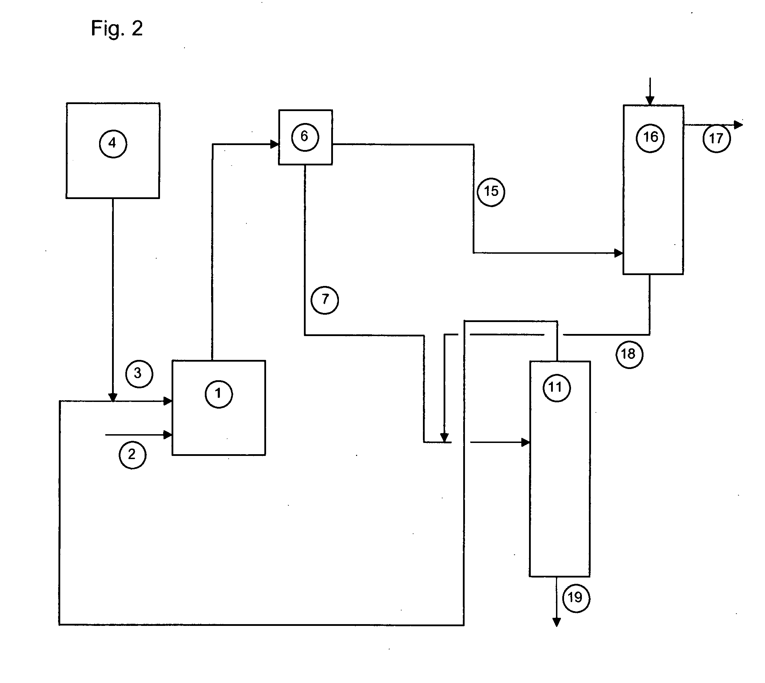

In einer Apparatur analog EP 428166 A1, Beispiel 1 wurde Chlormethan hergestellt. Die Apparatur ist in Fig. 2 illustriert.

In den heizbaren Reaktor (1), der Katalysator gelöst in Salzsäure enthält, wurden über Leitung (2) 283 1/h Si-Verbindungen enthaltender Chlorwasserstoff und über Leitung (3) 520 g/h Methanol aus der Methanolvorlage (4) und der Destillationskolonne (11) zugespeist.

Das im Prozess eingesetzte HCl-Gas stammte aus einer Anlage zur kontinuierlichen Hydrolyse von Dimethyldichlorsilan mit Salzsäure. Die Reinheit des Dimethyldichlorsilans betrug > 99,0 Gew.-%.
Der Gehalt an als Verunreinigung im HCl-Gas enthaltenen Si-Verbindungen, berechnet als Me₂SiO, betrug in Summe ca. 2000 ppm. Davon entfielen ca. 25 % auf die Cyclen D3, 50 % auf D4 und ca. 25 % auf D6. Daneben waren Spuren (< 50 ppm) von Dimethyldichlorsilan, alpha-omega Dichlorsiloxanen Cl(Me₂SiO)ₙCl mit n= 1-5, und Spuren von Verbindungen des Typs HO(Me₂Si)ₘX ; m= 1,2,3 ...; X= Cl, OH enthalten.
An nicht Si enthaltenen Verunreinigungen waren enthalten: verzweigte C7-Kohlenwasserstoffe (Trimethylbutane, Dimethylpentane, C7-Olefine) und Additionsprodukte von HCl an diese Olefine und Spuren Wasser.
Die Konzentrationsbestimmung der Verunreinigungen im HCl-Gas erfolgte gaschromatographisch.

Das den Reaktor (1) über Kopf verlassende Gemisch aus MeCl, MeOH, Spuren Dimethylether, HCl, Si-Verbindungen und Kohlenwasserstoffen wurde über Leitung (5) in einen mit Eiswasser gekühlten Kondensator (6) geleitet, auf ca. 10°C abgekühlt und das gebildete Kondensat gesammelt.

Das Kondensat (ca. 30 % Methanol, 6 % HCl; 63 % Wasser) enthielt 780 ppm Me₂SiO.

Messmethode: 1H-NMR (Lit: R. Lehnert; Nachr. Chem. Tech.Lab. 2009(09), 1167-1168).
Das Kondensat war durch die enthaltenen Si-Verbindungen leicht getrübt. Eine Phasenseparation trat jedoch auch nach 16 stündiger Lagerung nicht ein.
Das im Kondensator (6) abgeschiedene Kondensat wurde über Leitung (7) der Destillationskolonne (11) zugeführt.

Das aus dem Kondensator (6) entnommene Chlormethan gelangte über Leitung (15) in den Wasserwäscher (16). Dort wurden die beim Passieren des Kondensators (6)) nicht-kondensierten Bestandteile (MeCl, MeOH, DME, Wasser, HCl, Si-Verbindungen (Me₂SiO)) in einer mit Füllkörpern (Berlsättel aus Keramik 6X6 mm) gefüllten Kolonne (Innendurchmesser 50 mm, Höhe 1 m) im Gegenstrom mit 2000 g/h Wasser gewaschen. Eine Si-Bestimmung im MeCl nach dem Wasserwäscher (16) in der Leitung (17) ergab eine Me₂SiO Konzentration von 283 ppm.

Der Ablauf (18) des Wasserwäschers (16) aus einem Versuch von 5h Dauer (10,24 kg) wurde zunächst gesammelt. Der Ablauf enthielt Siloxane in Form von emulgierten fein verteilten Tröpfchen, die sich auch nach längerer Standzeit nicht durch Phasenseparation abtrennen ließen. Die Me₂SiO-Konzentration betrug 240 ppm. Der Ablauf enthielt ca. 2,5% Methanol. Der gesammelte Ablauf des Wasserwäschers (16) und das Kondensat des Kondensators (6) nach 5 h Versuchsdauer wurden gemischt (ca. 12kg) und zur Rückgewinnung von Methanol kontinuierlich in den unteren Teil einer beheizten Destillationskolonne (11), einer Füllkörperkolonne (1 m, 5 cm, Berlsättel aus Keramik 6X6 mm) dosiert. Der Sumpf bestand aus einem beheiztem 250ml Kolben mit Überlauf durch den der Füllstand konstant gehalten wurde. Nach Dosierung von ca. 5000g methanolischer Salzsäure mit einem Me₂SiO-Gehalt 310 ppm wurden in der Umgebung der Dosierstelle in der Kolonne Verlegung durch polymere Siloxane beobachtet. Der Versuch musste abgebrochen werden. Die Me₂SiO Konzentration im gewonnenen Destillat (ca. 70g; 10% Wasser, 90% MeOH) betrug ca. 880 ppm. Das Destillat der Destillationskolonne (11) wurde über Leitung (3) in den Reaktor (1) eingespeist. Der Sumpfablauf aus Leitung (19) war durch enthaltene Sx-Verbindungen getrübt. Es wurde eine Me₂SiO-Konzentration von 155 ppm gemessen.

Das Chlormethan verliess über Leitung (17) die Anlage.

**Tabelle 1: Bilanzierung von Me₂SiO in den Produktströmen einer MeCl-Synthese aus MeOH und HCl (g) mit einem Gehalt von 2000 ppm Me₂SiO nach 5 h Versuchsdauer**

| Bezugsziffer | Position | gemessen Me₂SiO in ppm | berechnet Me₂SiO in mg/h |
|---|---|---|---|
| (2) | HCl zu MeCl Reaktor (1) | 2000 | 910 |
| (7) | Kondensat | 780 | 238 |
| (18) | Ablauf Wasser-wäscher (16) | 243 | 498 |
| | Zulauf Destillations-kolonne (11) | 310 | 736 |
| | Kopf Destillations-kolonne (11) | 880 | 116 |
| (19) | Sumpf Destillations-kolonne (11) | 155 | 343 |
| (17) | gereinigtes MeCl | 283 | 171 |

87 % der über das HCl-Gas eingebrachten Si-Verbindungen gehen über das Abwasser oder als unerwünschte Verunreinigung im MeCl verloren. Der Gehalt an Si-Verbindungen in der als Nebenprodukt anfallenden methanolischen Salzsäure bereitete Schwierigkeiten bei der destillativen Aufarbeitung mit dem Ziel nicht umgesetztes Methanol zurückzugewinnen.
In der Me₂SiO-Bilanz der Methanolrückgewinnung ergab sich über 5h Versuchsdauer eine Fehlmenge von ca. 1 g = 45 % der eingebrachten Me₂SiO-Menge.

### Erfindungsgemäßes Beispiel 2

Das Verfahren wurde in einer Anlage gemäß Fig.1 durchgeführt. Das dazu eingesetzte HCl-Gas stammte aus einer Anlage zur kontinuierlichen Hydrolyse von Dimethyldichlorsilan mit Salzsäure. Die Reinheit des Dimethyldichlorsilans betrug > 99,0 Gew.-%.

Es wurden 506 l/h HCl mit 1070 g/h MeOH zu 485 l/h MeCl umgesetzt.
Die Me₂SiO-Konzentration im Ablauf des Methanolwäschers (13) betrug ca. 3500 ppm; (4,28 g/h). Die nach Abkühlung des Roh-MeCl im Kondensator (6) ablaufende Flüssigkeit wurde im Trennbehälter (8) gesammelt. Auf der Oberfläche schied sich eine Siloxanphase ab (Dichte der Si-Phase: 0,91-0,98g/cm³; 25°C). Die Menge der über Leitung (9) abgeschiedenen Siloxanphase betrug nach 24 h ca. 27 g. Die Me₂SiO-Konzentration in der wässrig-methanolischen Phase von Leitung (10) betrug 1340 ppm; (0,9 g/h). Die Phase war leicht getrübt. Die Güte der Phasentrennung kann über die Dichte der wässrigen metholischen Salzsäure beeinflußt werden.

Der Wasserwäscher (16) wurde wie im Beispiel 1 mit 2000g/h Wasserzulauf betrieben. Im MeCl (von) aus Leitung (17) nach dem Wasserwäscher (16) konnte kein Me₂SiO nachgewiesen werden. Im Ablauf (18) des Wasserwäschers (16) konnte ebenfalls kein Me₂SiO (< 50 ppm) nachgewiesen werden. Das ablaufende Wasser-Methanol-Gemisch war klar.
Der innerhalb von 24 h Versuchsdauer angesammelte Ablauf (18) des Wasserwäschers (16) und die methanolischen Salzsäure aus dem Trennbehälter (8) wurden gemischt und über die gleiche Destillationskolonne (11) wie in Beispiel 1 destilliert. Die Me₂SiO-Konzentration in der zugeführten Mischung (10) betrug 320 ppm.
Im Sumpfablauf der Destillationskolonne (11) wurden 225 ppm Me₂SiO (= 0,55 g/h) gefunden. Im Methanoldestillat (14) wurden 1000ppm; (0,35 g/h) Me₂SiO gefunden. In der Destillationskolonne (11) wurde kein polymeres Siloxan abgeschieden.

**Tabelle 2: Bilanzierung von Me₂SiO in den Produktströmen einer MeCl-Synthese aus MeOH und HCl(g) mit einem Gehalt von 2000 ppm Me₂SiO mit Me₂SiO-Abtrennung nach 24 h Versuchsdauer**

| Bezugsziffer | Position | Me₂SiO in ppm | Me₂SiO in mg/h |
|---|---|---|---|
| (2) | HCl zu MeCl Reaktor (1) | 2000 | 1600 |
| (7) | Kondensat | 1340 | 900 |
| (18) | Ablauf Wasserwäscher (16) | <50 | ~0 |
| (10) | Zulauf Destillations-kolonne (11) | 320 | 900 |
| (14) | Kopf Destillations-kolonne (11) | 1000 | 350 |
| (19) | Sumpf Destillations-kolonne (11) | 225 | 550 |
| (17) | gereinigtes MeCl | < 50 0 | ~0 |
| | Ablauf Methanolwäscher (13) | 3500 | 4300 |
| (9) | Sx-Kondensat aus Leitung (9) | pure | 1100 |

Von den mit dem HCl-Gas eingebrachten 1,6 g/h Me₂SiO konnten 1,1 g/h (68%) durch Phasentrennung aus dem MeOH-HCl-WasserGemisch im Trennbehälter (8) abgeschieden werden. Ca. 0,50g/h Me₂SiO gehen über den Sumpfablauf der Methanoldestillation als Abwasser verloren.
Die organische Phase aus dem Trennbehälter bestand zu ca. 90-99% aus Si-Verbindungen (hauptsächlich Cyclen D3 bis D10) Daneben waren enthalten, verschiedene, vor allem verzweigte C4-C12 Alkane, Alkene, Carbonylverbindungen, Chlorkohlenwasserstoffe, EtCl und MeCl. Das abgetrennte Si-Gemisch kann in verschiedener Weise verwertet werden:

Es kann beispielsweise ohne weitere Behandlung einem Reaktor zur Hydrolyse von Dimethyldichlorsilan zugespeist werden. Aus dem Si Gemisch können durch geeignete Verfahren (Destillation, Extraktion usw. ) enthaltene rein organische Verunreinigungen (wie zum Beispiel Alkane, Alkene, Carbonylverbindungen, Chlorkohlenwasserstoffe) abgetrennt werden und die dabei zurückbleibenden Si-Verbindungen erst danach durch Zuspeisung in den Reaktor zur Hydrolyse von Dimethyldichlorsilan verwertet werden. Man hat so die Möglichkeit unerwünschte Organika aus dem Verbund einer integrierten Anlage zur Herstellung von Siloxan abzutrennen.

## Patentansprüche

1. Verfahren zur Herstellung von Chlormethan aus Methanol und aus der Hydrolyse von Methylchlorsilanen stammendem mit Si-Verbindungen verunreinigtem gasförmigem Chlorwasserstoff, wobei die Si-Verbindungen ausgewählt werden aus Methylchlorsilanen, Methoxymethylsilanen und Hydrolyse- und Kondensationsprodukten daraus, bei dem aus dem gebildeten Chlormethan ein Teil der Si-Verbindungen auskondensiert werden und restliche Si-Verbindungen mit Methanol ausgewaschen werden und das dabei erhaltene Si-Verbindungen enthaltende Methanol zur Herstellung des Chlormethans mit Chlorwasserstoff eingesetzt wird und bei dem nach der teilweisen Auskondensation eines Teils der Si-Verbindungen aus dem Chlormethan eine Auftrennung des Kondensats in eine Phase der Si-Verbindungen mit organischen Verbindungen und eine Methanol-HCl-Wasserphase in einem Trennbehälter stattfindet.

2. Verfahren nach Anspruch 1, bei dem die abgetrennten Si-Verbindungen zurück in die Hydrolyse der Methylchlorsilane dosiert werden.

3. Verfahren nach Anspruch 1 oder 2, bei dem 30 bis 100 %, des im Verfahren eingesetzten Methanols zum Auswaschen der Si-Verbindungen eingesetzt werden.

4. Verfahren nach Anspruch 1 bis 3, bei dem die Umsetzung von Chlorwasserstoff mit Methanol zu Chlormethan in einer unter Reaktionsbedingungen flüssigen und Katalysator enthaltenden Phase durchgeführt wird.

5. Verfahren nach Anspruch 4, bei dem Aminhydrochloride als Katalysatoren eingesetzt werden.

## Claims

1. Process for the preparation of chloromethane from methanol and gaseous hydrogen chloride contaminated with Si compounds and originating from the hydrolysis of methylchlorosilanes, the Si compounds being selected from methylchlorosilanes, methoxymethylsilanes and hydrolysis products and condensates thereof, in which some of the Si compounds are removed by condensation from the chloromethane formed and remaining Si compounds are washed out with methanol and the methanol thus obtained and containing Si compounds is used for the preparation of chloromethane with hydrogen chloride and in which separation of the condensate into a phase of Si compounds with organic compounds and a methanol/HCl/water phase takes place in a separation container after the partial removal by condensation of some of the Si compounds from the chloromethane.

2. Process according to Claim 1, in which the Si compounds separated off are metered back into the hydrolysis of the methylchlorosilanes.

3. Process according to Claim 1 or 2, in which from 30 to 100% of the methanol used in the process are used for washing out the Si compounds.

4. Process according to Claims 1 to 3, in which the reaction of hydrogen chloride with methanol to give chloromethane is carried out in a catalyst-containing phase which is liquid under reaction conditions.

5. Process according to Claim 4, in which amine hydrochlorides are used as catalysts.

## Revendications

1. Procédé pour la production de chlorométhane à partir de méthanol et de chlorure d'hydrogène gazeux contaminé avec des composés siliciés, provenant de l'hydrolyse de méthylchlorosilanes, les composés siliciés étant choisis parmi les méthylchlorosilanes, les méthoxyméthylsilanes et les produits de condensation et d'hydrolyse de ceux-ci, dans lequel une partie des composés siliciés sont séparés par condensation d'avec le chlorométhane formé et les composés siliciés restants sont éliminés par lavage avec le méthanol, et le méthanol contenant les composés siliciés ainsi obtenu est utilisé pour la production du chlorométhane avec du chlorure d'hydrogène et dans lequel après la séparation par condensation partielle d'une partie des composés siliciés d'avec le chlorométhane a lieu une séparation du condensat en une phase des composés siliciés avec des composés organiques et une phase aqueuse de méthanol-HCl dans un récipient de séparation.

2. Procédé selon la revendication 1, dans lequel les composés siliciés séparés sont renvoyés dans l'hydrolyse des méthylchlorosilanes.

3. Procédé selon la revendication 1 ou 2, dans lequel 30 à 100 % du méthanol utilisé dans le procédé sont utilisés pour l'élimination par lavage des composés siliciés.

4. Procédé selon les revendications 1 à 3, dans lequel la réaction du chlorure d'hydrogène avec le méthanol conduisant au chlorométhane est effectuée dans une phase liquide dans les conditions réactionnelles et contenant un catalyseur.

5. Procédé selon la revendication 4, dans lequel des chlorhydrates d'amines sont utilisés comme catalyseurs.
